(19) Europäisches Patentamt
European Patent Office
Office européen des brevets

(11) **EP 3 799 051 A1**

(12) **EUROPEAN PATENT APPLICATION**

(43) Date of publication:
**31.03.2021 Bulletin 2021/13**

(51) Int Cl.:
**G16B 40/10** *(2019.01)*  **G16B 50/20** *(2019.01)*

(21) Application number: **19200381.2**

(22) Date of filing: **30.09.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Siemens Healthcare GmbH 91052 Erlangen (DE)**

(72) Inventors:
• **Frings, Oliver**
  **91058 Erlangen (DE)**
• **Krüger, Benedikt**
  **96250 Ebensfeld (DE)**
• **Kubala, Eugen**
  **80939 München (DE)**
• **Neumann, Dominik**
  **91052 Erlangen (DE)**
• **Würstle, Maximilian**
  **91083 Baiersdorf (DE)**

(54) **INTRA-HOSPITAL GENETIC PROFILE SIMILAR SEARCH**

(57)    Computer-implemented method for sharing medical information comprising the steps of receiving a first genomic data set, the first genomic data set being generated at a first site; comparing the first genomic data sets with a plurality of second genomic data sets stored in a database external to the first site; identifying, amongst the second genomic data sets, one or more reference genomic data sets, on the basis of determining a similarity between first genomic data set and the second genomic data sets; dispatching a notification (NOT) to the first site indicative of the one or more reference genomic data sets.

FIG 2

**Description**

In healthcare, physicians often base their decisions on experience on previous patient cases. The paradigm is that similar patients will respond similarly to the same treatment. Physicians therefore try to remember and associate similar patient cases to the one patient they currently care for in order to decide on further diagnostic procedures or on treatment options. Traditionally, the search for similar patients is up to the individual physician and therewith dependent on the physician's personal experience and network.

Recent years saw considerable effort in the healthcare business to automate and thereby objectify the search for similar patients. One approach in this regard is to automatically query databases for cases with similar diagnoses, similar medical findings and/or similar courses of diseases. While this certainly constitutes a promising first step, studies indicate that such criteria are often not specific enough to provide a reliable support for the physician. What is more, criteria such as prior diagnoses or findings are inherently subjective as well, as they are likewise based on human assessment.

What is therefore needed is an objective measure for the similarity between two cases. In principle, genetic data sets could provide such an objective standard of comparison. In oncology, the usage of large genetic data sets is a common approach in treating advanced cancer patients to decide on further treatment options with targeted therapies. However, the evidence for a lot of the mutations found in a patient's tumor is weak and their influence on therapy response is often unclear. Only rarely, the interpreting physician is able to use his/her knowledge of previous patients with similar genetic profiles to decide on a treatment option. This is due to the vast number of combinatorial mutations profiles and the little number of patients with a genetic tumor profile within one hospital. This has the consequence that much of the data available within one healthcare organization is generally sparse, and it is very difficult to determine, through manual searching, all of the relevant data that might be applicable to a particular patient. Accordingly, conventional clinical environments are not generally capable of matching patient information on the basis of genetic data sets.

For these reasons, it would be, in principle, desirable to extend the search for similar cases to incorporate a plurality of healthcare organizations. However, this is not straight-forwardly possible, as data privacy regulations impose tight constraints on the freedom to exchange medical information across different institutions. In particular, this applies for genetic data sets. For instance, it may be forbidden to directly exchange genetic raw data. For the same reasons, it is generally not possible to directly access genetic databases across different organizations and query them for similar cases.

Accordingly, it is an object of the present invention to provide means and/or methods which allow for an improved way of sharing medical information for similar patient cases. Particularly, it is an object of the present invention to provide means and/or methods that allow for a swift, objective and reliable identification of similar patient cases while respecting existing legal restrictions in exchanging medical information, and that allow for a seamless integration of the ensuing processes into existing clinical workflows.

This object is solved by a method for sharing medical data sets, corresponding system, corresponding computer-program product and computer-readable storage medium according to the independent claims. Alternative embodiments are object of the dependent claims and are set out below.

In the following, the technical solution according to the present invention is described with respect to the claimed apparatuses as well as with respect to the claimed methods. Features, advantages or alternative embodiments described herein can likewise be assigned to other claimed objects and vice versa. In other words, claims addressing the inventive method can be improved by features described or claimed with respect to the apparatuses. In this case, functional features of the method are embodied by objective units or elements of the apparatus, for instance.

According to a first aspect, a computer-implemented method for sharing medical information is provided. The method comprises several steps. A first step is directed to receiving a first genomic data set, the first genomic data set being generated at a first site. A further step is directed to comparing the first genomic data set with a plurality of second genomic data sets stored in a database external to the first site. A further step is directed to identifying, amongst the second genomic data sets, one or more reference genomic data sets, on the basis of determining a similarity between first genomic data set and each of the second genomic data sets. A further step is directed to dispatching a notification to the first site indicative of the one or more reference genomic data sets.

In other words, it is an idea of the present invention to base the search for similar cases on a comparison of genetic data sets. If there is a match between two genomic data sets, a corresponding notification is generated thereby sharing medical information. The matching involves the comparison with genomic data sets from a central knowledge database in which a plurality of genomic data sets is stored for comparison. The provision of a central database enables healthcare providers to upload genomic data sets to an external matching system which can more readily be configured to satisfy data privacy regulations when dealing with genomic data. In particular, by collecting genomic data in a central database, the access to the data can be tightly controlled while still enabling to exchange data. While healthcare providers may not be allowed to directly access external databases for retrieving similar patient cases, they may still send the genomic data sets to an external facility comprising the database and providing means for comparing and matching two genomic data sets.

A genomic data set generally relates to genomic data of a patient. Genomic data may, for instance, be obtained by a biopsy procedure involving extraction of sample cells or tissues for examination to determine the presence or extent of a disease by determining the genomic state. The genomic state may relate to the DNA or RNA sequence or the chromosomal state. In oncology, another common way of obtaining a genomic data set is to analyze liquid patient samples for tumor DNA/RNA and extract the corresponding DNA or RNA sequence and/or chromosomal state. The extraction of the genetic sequence from a patient sample may involve known techniques such as sequencing, genotyping, the usage of microarray platforms including RNA or mRNA expression, or the usage of polymerase chain reaction (PCR) platforms, copy-number variation (CNV) platforms, (whole) genome sequencing platforms or the like. Thus, first and second genomic data set may relate to raw genomic data such as the DNA and/or RNA sequences. Further, genomic data comprised in first and second genomic data set may be in the form of gene expression levels, gene states, chromosomal states or the like. What is more (and as will be further detailed below), first and second genomic data set may also relate to already processed genomic data of a patient. "Processed" may mean that one or more genomic features and/or characteristic values have been derived (i.e., extracted or calculated) from the genomic raw data (i.e., the gene sequence). The genomic features may relate to high-level information derived from the genomic data sets (as will be further detailed below). The genomic features may be selected or tailored according to the clinical question at hand. For oncology related questions, the genomic features may, for instance, rely on identifying mutations in the genomic data. Accordingly, corresponding genomic features might relate to the genomic regions of mutations in the genomic data sets, mutation hotspots in the genomic data sets, the effect of mutation in the genomic data sets (gain or loss), and/or the clinical actionability of mutations in the genomic data sets. Moreover, "processed" may mean that the genomic data underlying first and second genomic data sets underwent a filtering step. In this regard, information that does not identify a required piece of information such as a chromosomal DNA copy loss or gain may have been filtered out prior to forwarding the first genomic data set and/or storing the second genomic data sets in the database. As such, filtered genomic data may be created that generally only includes those regions of interest that may contain a chromosomal abnormality or alternation. In addition, first and second genomic data sets may comprise supplementary information such as information pertaining to the disease type and state of the patient, further patient information such as age or sex, the patient's health record, therapy and medication information, information about the practicing physician or the like. The supplementary information may be appended to the genomic data sets as metadata. Thus, summarizing the above, first and second genomic data sets may relate to raw or processed genomic data and may comprise metadata and supplementary information. Genomic data sets, may, for instance, comprise plain gene sequences, information about gene mutations, gene associations, gain or loss, gene expression levels or gene states or, in general, information about genomic testing.

The first site may be seen as relating to a first clinical organization or environment from where the first genomic data set originates. As such, the first site may be embodied by a hospital, clinical consortium of a plurality of hospitals, a practice, a gene or cancer center, gene laboratory or the like. In general, the second genomic data sets have not been generated at the first site, but at sites different than the first site (i.e., at other clinical organizations) and have been previously uploaded to the database from these other sites.

The database is a database of genomic information or a genomic knowledge database. It may include any storage medium or organizational unit for storing and accessing genomic data sets and any supplementary information associated with the second genomic data sets. The database may include a plurality of individual memory units and repositories and may, in particular, include distributed data architectures. The database may include a variety of data records accessed by an appropriate interface to manage delivery of genomic data sets and supplementary information. The database being "external" to the first site may mean that it is not within the premises of the first site. In other words, the database may be located at a site different from the first site. Noteworthy, the "location" of the database may also relate to a cloud platform, the server architecture of which is likewise external to the first site. The database may thus be seen as being physically separated from the first site. Further, it may be configured such that it cannot be accessed from the first site (or, generally, from the outside for that matter). The database may thus provide a platform for archiving sensible genomic information from a plurality of institutions (sites).

The step of comparing may comprise accessing the database and retrieving each of the stored second genomic data sets for comparison to the first genomic data set. However, the step of comparing may further comprise selecting a sub-group from the second genomic data sets for the ensuing identification of reference genomic data sets.

The step of identifying one or more reference genomic data sets is directed to identify those genomic data sets amongst the second genomic data sets that are similar to the first genomic data set. The similarity may amount to a plain similarity in gene sequences but may also include similar (higher-level) genomic features such as similar expression levels, similar gene mutation signatures, similar gain or loss, similar gene associations and so forth. Moreover, any of the available metadata (by ways of the supplementary information) may be factored in. For instance, the identification of similar genomic data sets may involve retrieving genomic data sets from patients of similar age, the same sex, and/or who underwent similar treatment. In other words, patient context information may be used to perform a matching process for identifying similar genomic data sets. In general, the step of identifying may comprise evaluating one or more similarity criteria. Mathematically, this may include extracting, from first and second genomic data sets one or more characteristic values according to the one or more similarity criteria, which characteristic values may then be compared to identify similar genomic data sets. The characteristic values may be aggregated to a score for each genomic data set, wherein individual characteristic values may be assigned different weights. Another expression for such procedure would be applying a similarity metric to the genomic data sets (which similarity metric comprises a plurality of similarity criteria). In other words, the step of identifying may comprise scoring first and second genomic data sets according to one or more similarity criteria (i.e., calculating a score for each genomic data set based on one or more similarity criteria). The similarity between two genomic data sets may be conceived as a "distance" between two genomic data sets in terms of one or more similarity criteria. The smaller the distance, the higher the similarity. If a score is calculated for each of the genomic data sets, the distance may be conceived as the difference between the scores of two genomic data sets. Another expression for "distance" would be "degree of similarity". Accordingly, the step of identifying may amount to identifying, amongst the second genomic data sets, reference genomic data sets having a degree of similarity to the first genomic data set above a certain value or threshold. The threshold for the degree of similarity (distance) may be seen as a figurative threshold. However, the step of identifying may likewise comprise setting a predetermined threshold in this regard (either automatically, semiautomatically or by a user). In addition, the threshold may be seen as an appropriate margin of similarity around one or more characteristic values determined for the first genomic data set for quantifying the similarity to other genomic data sets.

The notification to the first site notifies the first site that a reference genomic data sets has been found. It enables a user at the first site to initiate further steps in order to take advantage of that information. The notification may comprise additional information that allows the user to contact colleagues associated with the one or more reference genomic data sets. To this end, the notification may comprise an indication of the site of origin and/or the responsible physicians of the one or more reference genomic data sets. The notification may comprise the therapy and treatment response, genetic tumor profile corresponding to the reference genomic data set. The notification may be dispatched via a dedicated communication channel. The dedicated communication channel may be further configured to permit direct communication between the respective physicians, for instance, by exchanging text messages or by setting up telephone and/or video conferences. Further the notification may contain a link (e.g., in the form of an URL) for one-time access to the reference genomic data sets and the corresponding supplementary information in the database.

The steps according to the first aspect preferably happen external to the first site. In other words, the steps of receiving, comparing, identifying, and dispatching are carried out externally to the first site. These steps may be complemented by corresponding steps happening at the first site. These steps may comprise uploading the first genomic data set (to the database or corresponding system external to the first site) and receiving the notification. Further optional steps happening at the first site may be: generating the first genomic data set, selecting the first genomic data set for upload, and/or pre-processing the first genomic data set prior to uploading it. Of note, these steps may likewise form part of the method according to the first aspect.

In summary, the above steps synergistically contribute to an improved way of automatically finding similar cases and thereby facilitate an efficient exchange of medical information for similar patient cases. Specifically, the usage of genomic data sets for identifying similar cases introduces an objective measure for matching similar cases. This is because the genomic data sets as such do not depend on subjective diagnosis steps. The usage of a database which collects comparative genomic data sets across a plurality of institutions (sites) enables to considerably increase the amount of comparative data. Since the number of combinatorial similarity criteria in connection with genomic data sets is huge, the clustering of comparative data from a plurality of institutions is one of the preconditions for efficiently using genomic data sets for similar patient searches. What is more, the automated comparison and identification of similar cases according to the above aspect greatly facilities the procedure as any manually searching can be dispensed with. Moreover, the usage of the central database as a platform for identifying similar cases provides a way of sharing medical information in highly regulated environments. Through the intermediation of the database it is not required to directly exchange genomic data sets between institutions and/or to grant direct accesses to local databases storing sensible patient information. The usage of a central database is complemented with a notification step which informs participating users of similar patient cases and at the same time enables to channelize and regulate the information content forwarded to the users. In particular, this allows to provide meaningful feedback about similar patient cases and at the same time ensures that the procedure is in line with all relevant data privacy regulations. What is more, the method according to the first aspect readily integrates into clinical workflows, as the actual process steps are outsourced and performed automatically.

According to an embodiment, the method further comprises the step of introducing (or adopting) the first genomic data set in the database.

The step of introducing archives the first genomic data set in the database. With that, the first genomic data set may be used as comparative genomic data set (i.e., second genomic data set) for future cases. Introducing may further comprise storing any supplementary information provided together with the first genomic data set. As mentioned, the supplementary information may be appended to the genomic data set (as metadata) or provided in the form of separate files. Upon receipt, the first genomic data set may be assigned a unique identifier and all supplementary information may be assigned the same unique identifier unambiguously linking it to the respective first genomic data set. The unique identifier may be an accession number or any other suitable electronic identifier.

By including the first genomic data set (and any supplementary information) into the database alongside the second genomic data sets, the shared knowledge comprised in the system is enhanced and the similar patient search is rendered more efficient for subsequent queries.

According to an embodiment, first and/or second genomic data sets are anonymized, or, in other words, do not comprise any personal information pertaining to the patient.

"Anonymized" may mean that first and second genomic data sets do not reveal or contain any information from which the patient can be identified (i.e., patients name, address, photographs and the like). According to an embodiment, the method may further comprise the step of anonymizing the first genomic data set. The step of anonymizing may comprise filtering out any personal information with which the patient can be identified. The step of anonymizing may be carried out either at the first site or upon receiving the first genomic data set, i.e., external to the first site.

By anonymizing the genomic data sets, it can be safely ruled out that the information contained in the genomic data sets or in the associated supplementary information can be traced back to the corresponding patient.

According to an embodiment, the database is a local database located at a second site different than the first site. Consequently, the first genomic data sets are received at the second site, and the steps of comparing, identifying and dispatching are carried out at the second site.

In other words, this embodiment covers an implementation according to which the database sits at a local healthcare organization which provides its services to other institutions. In this respect, the database is a local database within the premises of the second site. Such a configuration may be beneficial if the access to the database needs to be tightly controlled. For instance, the interface to the database may be configured such that the database can only be accessed from within the second site without any direct connection to external networks. Like the first site, the second site may be a hospital, clinical consortium of a plurality of hospitals, a practice, a gene center or the like. The second genomic data sets contained in the database may either stem exclusively from the second site or originate from a plurality of external sites.

According to an embodiment, the database is configured as a cloud platform and the first genomic data sets are received at the cloud platform with the steps of comparing, identifying and dispatching being carried out at the cloud platform. The embodiment constitutes a second exemplary implementation of the database. Implementing the database as a cloud platform has the advantage that it can be more readily accessed from the sites participating in the patient similarity search program. Further, the entire communication between the individual sites (e.g., once a reference genomic data set has been found) may then be routed via the cloud platform. This may reduce the operational burden at the local sites and may decrease the hurdle for the local sites to participate. In turn, this may have the benefit that the build-up of the knowledge database is fostered. At the same time, data confidentiality may still be maintained by configuring the cloud platform such that the database cannot be directly accessed from the outside.

According to an embodiment, the step of identifying is based on applying a trained function to the first genomic data set. According to a further embodiment, the step of identifying is based on applying the trained function to first and second genomic data sets.

A trained function maps input data to output data. The output data can, in particular, depend on one or more parameters of the trained function. The one or more parameters of the trained function can be determined and/or be adjusted by training. The determination and/or the adjustment of the one or more parameters of the trained function can be based, in particular, on training data. The training data may comprise a pair made up of training input data and associated training output data. For creating training mapping data, the trained function is applied to the training input data. In particular, the determination and/or the adjustment can be based on a comparison of the training mapping data and the training output data.

Other terms for trained function are trained mapping specification, mapping specification with trained parameters, function with trained parameters, algorithm based on artificial intelligence, algorithm of machine learning. An example for a trained function is an artificial neural network, wherein the edge weights of the artificial neural network correspond to the parameters of the trained function.

In particular, the trained function may be applied to at least the first genomic data set. Additionally, the trained function may be applied to the second genomic data set. The trained function may be applied to the first genomic data set upon receipt of the first genomic data set. The trained function may be applied to the second genomic data set upon identifying the reference genomic data set or already prior to that, in particular, already (long) before the first genomic data set is received. The trained function may be trained to output genomic features and/or characteristic values. The corresponding outputs of the trained function may then be stored in the database alongside or in lieu of the corresponding second genomic data sets. According to some implementations, the trained function is applied to the genomic data sets upon storing them in the database.

The trained function may be configured (trained) so as to output a similarity score for the first genomic data set which can be matched with corresponding similarity scores of the second genomic data sets upon identifying the one or more reference genomic data set. The trained function may be further configured (trained) to output one or more genomic features and/or characteristic values of the first genomic data set which can be compared to corresponding genomic features and/or characteristic values of the second genomic data sets upon identifying one or more reference genomic data set. Accordingly, the corresponding outputs of the trained function may be seen as providing "intermediate results" on the basis of which the one or more reference genomic data set may be identified. Of note, the further processing of the intermediate results may likewise be based on applying the same or another trained function to the intermediate results. Further, the trained function may be configured (trained) to directly identify the one or more reference genomic data sets when applied to the first genomic data set (i.e., without outputting intermediate results). However, the usage of intermediate results may be beneficial to reduce the amount of data that needs to be stored and exchanged. Further, the usage of intermediate results may be beneficial from the perspective of data confidentiality. This is because the genomic data set can be effectively stripped from any genomic raw data by extracting genomic features and/or characteristic values. If the trained function is provided to the first site, genomic features and/or characteristic values may be calculated on-site. This opens the possibility to forward this information in the first genomic data set in lieu of the raw data.

In the training phase, the trained function may be trained on appropriate training data. The training data may comprise test genomic data sets as training input data and reference genomic data sets as training output data the similarity of which has been verified (e.g., by humans).

The usage of a trained function for identifying one or reference genomic data sets has the advantage that the trained function may learn to rely on features, characteristics, and insights for quantifying the similarity of two genomic data sets which are not readily accessible by traditional techniques and/or the human mind. Moreover, using trained functions for identifying one or more reference genomic data sets enables a fast, i.e., basically on-the-flight search of a high number of second genomic data sets stored in the database. Further, the usage of trained functions synergistically contributes to the requirement of keeping genomic data as confidential as possible. This is because the usage of trained functions facilitates a highly autonomous data processing scheme requiring no or only little interactions with human operators (which might breech data confidentiality). Moreover, the trained function can be readily configured not to output any sensible personal information. Thus, the trained function may also be used to anonymize genomic data sets.

According to an embodiment, the trained function is based on a support vector machine algorithm and/or a random forest algorithm and/or a regularized regression model.

Support vector machine algorithms, random forest algorithms as well as regularized regression models have proven particularly versatile in classifying data sets in general. Moreover, these algorithms showed particularly good results in connection with the analysis of genetic information. In extensive tests, the inventors have recognized that these algorithms are particularly suited for matching genomic data sets in similar patient searches.

According to an embodiment, first and second genomic data sets comprise supplementary information or metadata associated to the genetic information and the step of identifying is based on the supplementary information or metadata.

The supplementary information or metadata may comprise patient context information. Such context information may include information pertaining to a disease state of a particular patient, age, sex, or patient history. Further, the supplementary information or metadata may comprise disease phenotypes and genetic alterations. As such, the supplementary information may be factored in in the process of identifying one or more reference genomic data sets. For instance, in the step of identifying, the search may be focused on genomic data sets from patients with similar disease phenotypes, in the sense that these genomic data sets are preselected for further detailed analysis. This has the benefit, that the performance of the similarity search may be increased both in terms of accuracy and speed. Likewise, the trained function may use the supplementary information as further input data. According to an embodiment, first and second genomic data sets comprise supplementary information and/or metadata associated to the genetic data sets and the step of comparing comprises preselecting the second genomic data sets on the basis of the supplementary information and/or metadata.

Preselecting may, for instance, comprise sorting genomic data sets with matching metadata into one or more groups. In the ensuing step of identifying only such genomic data sets may be considered that fall in the same group as the first genomic data set. According to an example, the aforementioned groups may relate to disease groups of cases having a clinical and functional similarity of the underlying diseases. Such disease groups may relate to grouping the genomic data sets according to tumor types, for instance. In a similar manner, alterations may be grouped into alteration groups that are functionally similar.

According to an embodiment, the first and or second genomic data sets comprise one or more genomic features respectively derived from an underlying genetic sequence of a patient, and the step of identifying is based on the one or more genomic features.

A genomic feature is a feature that has been calculated and/or extracted from genetic raw data such as the gene sequence. Thus, the genomic feature may be seen as high-level representation of one or more characteristics encoded in a gene sequence. In other words, genomic features are data objects extracted from the gene sequence. The genomic features may be associated to the aforementioned similarity criteria, preferably such that each genomic feature corresponds to similarity criteria. Generating the genomic features may comprise processing the first and second genomic data sets so as to respectively extract, from the first and second genomic data sets, one or more genomic features, respectively corresponding to the one or more similarity criteria. In contrast to the aforementioned characteristic values, genomic features relate to more abstract data packages or objects. As such, genomic features may comprise different kinds of information from sequence excerpts to gene expression profiles to plain numbers. Genomic features may thus be seen as containers for transporting arbitrary higher-level information about a gene sequence. Genomic features may be related to the characteristic values. On the one hand, a genomic feature may be a characteristic value by itself (if, for instance, the genomic feature relates to a number). On the other hand, one or more characteristic values may be derived from a genomic feature by further processing. Examples for genomic features may be annotated functions associated to a genetic region. An example would be a protein coding gene. Further genomic features may in general address information about mutations in the gene sequence. This may include the location/existence of mutation hotspots in the genomic data sets as one genomic feature (hotspots are regions in a genome that exhibit elevated rates of mutations relative to a neutral expectation), the effect of a mutation as further genomic feature or the clinical actionability of mutations as yet a further genomic feature. For instance, such genomic features may be output by the trained function (e.g., in the form of the aforementioned intermediate results).

The usage of genomic features constitutes a way to condensate the relevant information for conducting similarity search based on genomic data. This is beneficial in terms of the system requirements for exchanging and storing genomic data sets. In addition, the process of identifying reference genomic data set may be rendered more efficient since a smaller amount of data needs to be digested. Moreover, the usage of genomic features also contributes to the data privacy. This is because (although being of course based on gene sequences) genomic features preferably do not contain any dedicated (whole) gene sequence. While the gene sequence constitutes a genetic fingerprint from which a corresponding patient can be identified, this is no longer possible (or at least considerably more difficult) for genomic features.

Therefore, according to an embodiment, first and/or second genomic data sets consist of one or more genomic features. Preferably, they do not contain any explicit gene sequences anymore.

Upon identifying one or more reference genomic data sets, each individual genomic feature may be individually compared. Alternatively, identification may be based on a condensed feature parameter (also denoted as a genomic feature set or genomic feature vector) which is based on a plurality of individual genomic features. According to an embodiment, first and second genomic data sets thus comprise a feature vector of a plurality of individual genomic features.

According to an embodiment, the one or more genomic features comprised in the first genomic data set are generated at the first site.

According to the above explanations, the usage of genomic features enhances the performance of the method, limits the amount of exchanged data and contributes to the data security. In this regard, deriving the genomic features already at the first site makes it possible to only forward high-level features. Genomic raw data, from which a patient may still be identified, may be retained on-site.

According to an embodiment, the step of identifying comprises extracting on or more genomic features from the first genomic data set.

The extraction may be performed at the first site prior to forwarding the first genomic data set or after receipt of the first genomic data set, e.g., at the cloud platform or at the second site. The extraction may be performed by applying the trained function to the first genomic data set.

According to an embodiment, the step of identifying comprises determining a similarity between the first genomic data set and the second genomic data sets by comparing the one or more genomic features of the first genomic data set to the corresponding one or more genomic features of the second genomic data sets.

According to an embodiment, the step of identifying comprises comparing a genomic feature vector of the first genomic data set to a corresponding genomic feature vector of the second genomic data sets.

According to an embodiment, the first and second genomic data sets each comprise a genomic feature vector being respectively generated from corresponding raw gene sequences (optionally by respectively applying a trained function to the raw gene sequences), wherein in the step of identifying, the similarity between first and second genomic data sets is estimated based on a comparison of their corresponding genomic feature vectors.

According to an embodiment, the step of identifying comprises: determining one or more similarity criteria associated with the first and second genomic data sets, processing the first and second genomic data sets so as to respectively extract, from the first and second genomic data sets, one or more characteristic values respectively corresponding to the one or more similarity criteria, and identifying the one or more reference genomic data sets on the basis of the characteristic values.

Characteristic values may in general be characteristic numbers which alone or as an ensemble classify or identify a genomic data set, e.g., for comparing it to others but also for compressing the amount of data contained in a genomic data set for storing or data exchange. Each characteristic value may relate to a similarity criterion usable for retrieving the one or more reference genomic data set. Each characteristic value may correspond to one genomic feature as introduced above. Accordingly, the characteristic values may likewise be calculated from the genetic raw data, e.g., by applying a trained function to the raw data. Moreover, characteristic values may also relate to metadata such as patient's sex, age, or treatment response and so forth. As mentioned, the step of processing for extracting the characteristic values may take place already at the first site - with the benefit that only the characteristic values need to be forwarded (thereby reducing the amount of data exchanged and increasing the data security).

Determining the similarity criteria may involve choosing or adapting the similarity criteria according to the first genomic data set currently under consideration. Further, determining may relate defining a plurality of standardized criteria according to which each genomic data set is processed by default.

Noteworthy, the first and second genomic data sets may be processed independently from one another. In particular, the second genomic data sets may be processed before or long before the receipt of the first genomic data set. Specifically, the second genomic data sets' characteristic values may already be comprised in the second genomic data sets as stored in the database - either alongside or in lieu of any genetic raw data. As explained, the latter variant is beneficial in terms of storage space and data security.

According to an embodiment, the processing of the first genomic data set so as to extract, from the first data set, the one or more characteristic values is performed at the first site.

This has the effect that only the characteristic values and no raw data need to be forwarded by the local sites. As mentioned, this is beneficial in terms of data confidentiality and contributes to lowering the amount of data that needs to be exchanged.

According to an embodiment, one or more (or all) similarity criteria (and therewith the corresponding characteristic values) are based on an evaluation of gene mutations.

As regards oncology related questions, focusing on mutations in genomic data bears several advantages. On the one hand mutations allow for an efficient identification of reference genomic data sets since mutations usually pinpoint a disease or disease state very well. Further, characteristic values associated with mutations may furthermore be useful for physicians to evaluate the case at hand, e.g., in molecular tumor boards.

Specifically, the similarity criteria may comprise genomic regions (areas in the gene sequence) of mutations in the genomic data sets, mutation hotspots in the genomic data sets (hotspots are regions in a genome that exhibit elevated rates of mutations relative to a neutral expectation), mutation consequences in terms of gain and/or loss of function, effects of mutations on the signaling pathway, the clinical actionability of mutations in the genomic data sets, tumor profiles, disease types, patient's age and/or sex, treatment plan and/or treatment response and any combination thereof. In turn, the corresponding characteristic values are based on and are indicative of these criteria.

The clinical actionability is, in other words, a measure of whether clinical action should be taken based on heterogeneous information generated by genomic analysis. As regards the clinical actionability, the ESMO Scale for Clinical Actionability of molecular Targets (ESCAT) may be used, for instance. Alternatively, the clinical actionability may be determined according to the guidelines of the Association for Molecular Pathology (AMP).

The above characteristics have proven useful for the process of identifying similar cases on the basis of comparing genomic data sets. Moreover, these values enable an efficient data exchange in regulated environments. On the one hand, this is because they are uncoupled from the underlying gene sequences (which might still allow to identify the patient). On the other hand, values according to the above criteria provide indices anyway relevant for deciding on a case.

According to an embodiment, the step of identifying comprises calculating, for the first and second genomic data sets, a score as the weighted sum of the respective characteristic values, and comparing the scores of first and second genomic data sets.

By introducing a weighting of the individual characteristic values, in other words, different similarity criteria may be weighted differently for identifying the reference genomic data set. With that, different criteria may be balanced that contribute differently to the degree of similarity between two genomic data sets. According to an embodiment, the weights comprised in the weighted sum may be provided by the trained function.

According to an embodiment, the similarity between the first genomic data set and a second genomic data set is proportional to the difference in scores between the first and second genomic data sets. According to a further embodiment, the identification of the reference genomic data sets amongst the second genomic data sets may involve selecting those seconding genomic data sets as reference genomic data sets the score of which corresponds to the score of the first genomic data set within a predetermined margin. The predetermined margin may be set automatically and/or (semi-)automatically and/or by a user.

According to an embodiment, the step of identifying comprises generating a ranking of the reference genomic data sets on the basis of their similarity to the first genomic data set. The ranking may be based on the aforementioned difference in scores, the characteristic values, the genomic features or any of the explained similarity criteria. By ranking the reference genomic data set, the first site may be provided with an indication as to the relevance of retrieved reference genomic data set. The higher a reference genomic data set is ranked, the more relevant it might be for the case at hand. In doing so, the method effectively integrates into existing workflows and helps the involved physicians to focus on the most relevant information.

According to an embodiment, the step of dispatching further comprises the step of retrieving, for each reference genomic data set, supplementary information, and including the supplementary information in the notification.

As mentioned, the supplementary information may be stored alongside the second genomic data sets in the same or a different database. The supplementary information may be retrieved based on appropriate unique identifiers respectively assigned to each genomic data set stored in the database and the corresponding supplementary information. By including the supplementary information, the first site may be provided with additional information relevant for the case and not already provided in the notification.

According to an embodiment, the supplementary information comprises contact information associated to the reference genomic data sets, an information at which sites the reference genomic data sets have been generated, a therapy history associated to the reference genomic data sets, a treatment response profile associated to the reference genomic data sets a genetic tumor profile associated to the reference genomic data sets, and any combination thereof.

By providing the first site with an information about the site of origin and/or the treating physician of the respective reference genomic data set, a physician at the first site is enabled to retrieve additional information about the respective reference genomic data set and consult with her or his colleagues. As this involves forwarding personal data about the physician and not about the patient, the patient's data confidentiality is maintained. Likewise, the genetic tumor profile is of immediate use for the physicians at the first site as it provides valuable insights at one glance and can be readily discussed at the tumor boards at the first site. Further, since the tumor profile cannot be traced back to the patient, data confidentiality is maintained also with respect to this piece of information. The same holds true for the (anonymized) treatment history and treatment response profiles, which enable a treating physician to figure out which therapeutic measures have proven useful in parallel cases. To further ensure data privacy, the step of dispatching may comprise a step of anonymizing the notification such that it does not reveal or contain any information from which the patients belonging to the one or more reference genomic data set can be identified (i.e., patients name, address, photographs and the like).

According to a further embodiment, the notification includes the one or more reference genomic data sets.

For data security reasons, the reference genomic data sets included in the notification preferably do not contain any genetic raw data such as gene sequences but only high-level information that cannot be traced back to the respective patient (such as the aforementioned characteristic values, genomic features, similarity criteria or scores). To this end, an additional step of filtering the reference genomic data set may be provided before appending them to the notification striping the reference genomic data set from any genetic raw data.

According to an embodiment, the step of dispatching comprises including the one or more characteristic values of the first genomic data set and/or the corresponding one or more characteristic values of the respective reference genomic data set into the notification.

With that, the physician at the first site may be provided with meaningful information as to why a respective reference genomic data set has been chosen and where the similarities and differences lie. Further, dependent on the underlying similarity criterion, the information therewith provided may be useful for the further analysis of the case.

According to an embodiment, the method further comprises the step of establishing a communication channel for direct communication between the first site and the respective sites of origin of the one or more reference genomic data sets.

The communication channel constitutes an interactive connection between the matched sites. The communication channel may enable real-time interaction between the treating physicians, e.g., by exchanging voice or text messages. The communication channel may be embodied in the form of a chatroom or virtual molecular tumor board, e.g., hosted by the cloud platform or the aforementioned second site. The communication channel may be based on a secured connection. The communication channel may be based on a VPN connection. Providing the communication channel may comprise a log-in step for the treating physicians using a registered ID and password which may be forwarded in the notification or via a separate communication channel such as via email or sms ("short message service"). Access to the communication channel may be provided by an URL included in the notification or via existing user accounts. Information between participants may be exchanged in the form of verbal and/or written or textual communication. As such, the communication channel may be embodied by secured internet connection, preferably comprising a voice over internet protocol (VoIP) connection and/or a (text/video or audio) chat connection. The communication channel may also provide for graphical user interfaces at the matched sites, e.g., in the form a web client.

According to an aspect, a system for sharing medical information is provided. The system comprises an interface unit, a database and a computing unit. The interface unit is configured to communicate with a first site for receiving a first genomic data set. Further the interface unit is configured to communicate with the database. The database is configured to store a plurality of second genomic data sets, the database being external to the first site. The computing unit is configured to compare the first genomic data sets with a fraction or all of the second genomic data sets and to identify, amongst these second genomic data sets, one or more reference genomic data sets, on the basis of determining a similarity between first genomic data set and the respective second genomic data sets. Further, the computing unit is configured to dispatch a notification to the first site indicative of the reference genomic data sets via the interface unit.

The interface unit may be understood as an interface for data exchange at least between the first site, the system and any other sites of origin of the second genomic data sets. To this end, the interface unit may be configured to communicate over one or more connections or buses. The interface unit may be embodied by a gateway or other connection to a network (such as an Ethernet port or WLAN interface). The network may be realized as local area network (LAN), e.g., an intranet, ethernet or a wide area network (WAN), e.g., the internet. The network may comprise a combination of the different network types. According to an embodiment, the network connection may also be wireless.

The computing unit can be realized as a data processing system or as a part of a data processing system. Such a data processing system can, for example, comprise a cloud-computing system, a computer network, a computer, a tablet computer, a smartphone and the like. The computing unit can comprise hardware and/or software. The hardware can be, for example, a processor system, a memory system and combinations thereof. The hardware can be configurable by the soft-ware and/or be operable by the software. Generally, all units, sub-units or modules may be at least temporarily be in data exchange with each other, e.g. via network connection or respective interfaces. Consequently, individual units may be located apart from each other, especially the definition unit may be located apart, i.e. at the mobile device, from the remaining units of the computing units.

According to an embodiment of the present invention, the system is adapted to implement the inventive method for sharing medical information. The computing unit may be seen as a matching engine configured to compare the received first genomic data set to the second genomic data sets stored in the database and identify one or more reference genomic data sets on that basis. To this end, the computing unit may be configured to access the database and retrieve one or more second genomic data sets for comparing them with the first genomic data set. Further, computing unit may be configured to process the first genomic data set and/or the second genomic data sets for identifying one or more reference genomic data sets. The processing may comprise extracting one or more genomic features respectively from first and second genomic data sets, calculating one or more characteristic values respectively from first and second genomic data sets, respectively calculating a score for first and second genomic data sets, and calculating a degree of similarity between first and second genomic data sets (on the basis of one or more of the aforementioned processing steps). Further, the computing unit may be configured to rank the identified reference genomic data sets according to their similarity to the first genomic data set. The computing unit may further be configured to run a trained function (to apply a trained function to the first and second genomic data sets) in the step of identifying one or more reference genomic data set. Further, the computing unit may comprise communication modules configured to initiate and/or control the communication between the first site and the sites of origin of the one or more reference genomic data sets. To this end, the communication modules may be configured to dispatch a notification to the first site that one or more reference genomic data sets have been found, e.g., via the interface unit or any other appropriate channel. Further, the communication modules may be configured to establish a communication channel between the first site and sites of origin of the one or more reference genomic data sets. The communication channel may be hosted by the system, e.g., via the communication modules and/or the interface, so that any information exchange is routed through the system. As an alternative, the communication channel may be configured as a direct communication channel between the involved sites.

The system may be configured as a local system characterized in that all system components (i.e., databases, computing and interface units) are arranged at one defined local site, such as a hospital, cancer or gene center. Although the system components may still be spread throughout the local site, e.g., in the form of a local server architecture, all processes run on premises within the local sites and all databases and repositories are likewise arranged within the local site.

As an alternative, the system may be configured as a cloud system or cloud platform comprising a real or virtual group of computers and database like a so called 'cluster' or 'cloud'.

According to an aspect, a computer program product is provided. The computer program product comprises program elements which induce a computing unit of a system for sharing medical information to perform the method as described above in connection with one or more embodiments, when the program elements are loaded into a memory of the computing unit.

According to a further aspect, program elements are stored that are readable and executable by a computing unit of a system for sharing medical information, in order to perform steps of the as described above in connection with one or more embodiments, when the program elements are executed by the computing unit.

The realization of the invention by a computer program product and/or a computer-readable medium has the advantage that already existing providing systems can be easily adopted by software updates in order to work as proposed by the invention.

The computer program product can be, for example, a computer program or comprise another element next to the computer program as such. This other element can be hardware, for example a memory device, on which the computer program is stored, a hardware key for using the computer program and the like, and/or software, for example a documentation or a software key for using the computer program. The computer program product may further comprise development material, a runtime system and/or databases or libraries. The computer program product may be distributed among several computer instances.

In summary, by providing a platform for securely storing comparative data and processing uploaded genomic data sets, the invention establishes a way to base patient similarity search on genomic data and securely exchange information across a plurality of involved local sites.

Characteristics, features and advantages of the above described invention, as well as the manner they are achieved, become clearer and more understandable in the light of the following description and embodiments, which will be described in detail with respect to the figures. This following description does not limit the invention on the contained embodiments. Same components or parts can be labeled with the same reference signs in different figures. In general, the figures are not drawn to scale. In the following:

FIG 1 depicts a system for sharing medical information according to an embodiment,
FIG 2 depicts a system for sharing medical information according to another embodiment,
FIG 3 depicts a flowchart illustrating a method for sharing medical information according to an embodiment, and
FIG 4 depicts a flowchart illustrating a method for sharing medical information according to an embodiment.

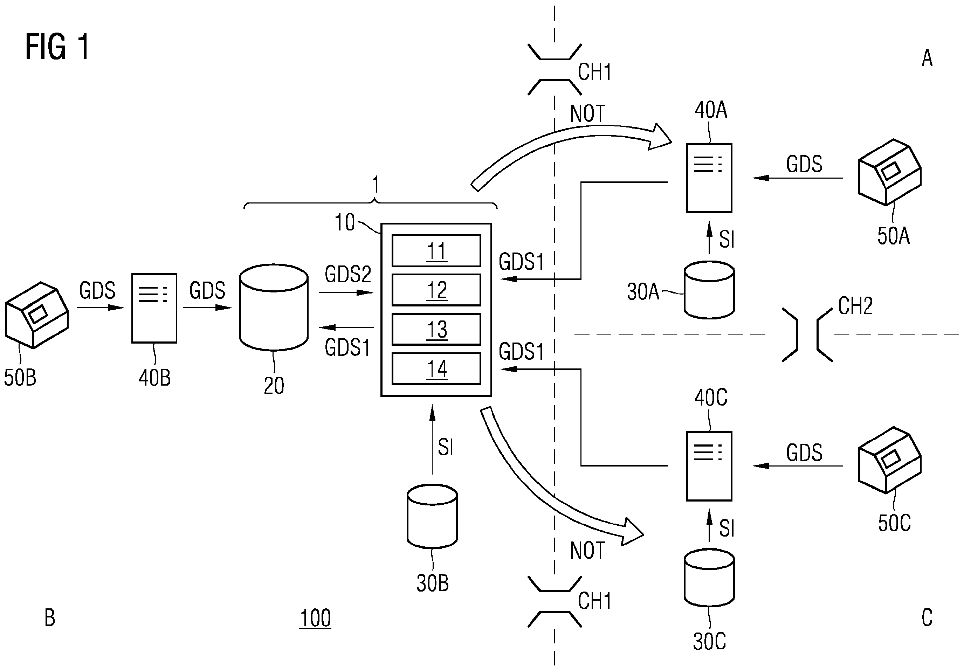

**Figure 1** depicts a distributed environment 100 for sharing medical information based on genomic similarities between patients according to an embodiment. Distributed environment 100 comprises a matching system 1 for sharing medical information (also denoted as "system") and two or more local sites A, B, C. The local sites may relate to medical or clinical environments such as hospitals, laboratories, gene centers, cancer centers or the like. In the example, three local sites A, B, C are shown for illustration. Distributed environment 100 is not limited to this number, however. In general, distributed environment 100 may comprise any number of local sites A, B, C.

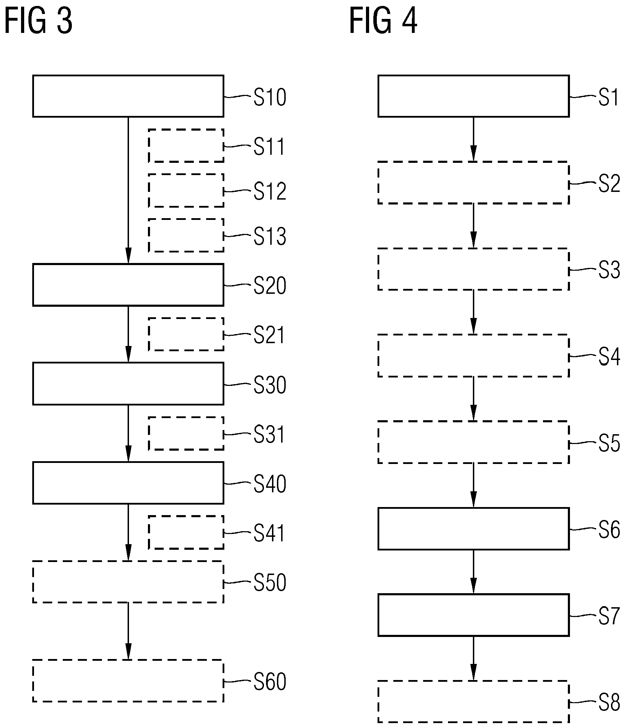

Local sites A, B, C may contain local computing units 40A, 40B, 40C through which one or more users (such as physicians or other healthcare personnel) may interface to the system 100. Local computing units 40A, 40B, 40C may comprise a hardware or software component, e.g., a micro-processor or a FPGA ('Field Programmable Gate Array). Local computing units 40A, 40B, 40C may be embodied as workstations, tablets, smart phones, server systems or connectivity nodes. Local computing units 40A, 40B, 40C may be configured to perform steps according to the workflow described in connection with Figures 3 and 4.

Further, local sites A, B, C may contain acquisition units 50A, 50B, 50C for acquiring genomic data and transferring the genomic data into genomic data sets GDS. The genomic data acquired may be raw data or already processed genomic data. Raw data may be acquired from acquisition units including but not limited to microarray platforms including RNA or mRNA expression, genotyping, gene expression platforms, polymerase chain reaction (PCR) platforms, copy-number variation (CNV) platforms, (whole) genome sequencing platforms or the like. The genomic data acquired from the acquisition unit 50A, 50B, 50C may be in the form of gene sequences, gene expression levels, gene states or the like. Alternatively, the acquisition may be from storage or memory, such as acquiring a previously created genomic data set GDS from an appropriate archiving system as acquisition units. The raw data may subsequently be processed or (pre-)processed in the acquisition units 50A, 50B, 50C and/or in the local computing units 40A, 40B, 40C.

To interface with one or more users, local computing units 40A, 40B, 40C may comprise a user interface such as one or displays or touch screens. Local computing units 40A, 40B, 40C may be configured as reading workplaces with which users can retrieve and review genomic data sets GDS and related supplementary information SI. To retrieve the supplementary information SI, local computing units 40A, 40B, 40C may be configured to query appropriate local data storage devices or repositories 30A, 30B, 30C within the respective sites A, B, C, for instance. To this end, local computing units 40A, 40B, 40C may be configured to extract a unique identifier from the genomic data sets GDS, indicative of the patient or case under consideration. Such unique identifier may be a patient ID, a case or accession number, a patient name or the like. The unique identifier may be assigned to the genomic data sets GDS upon their acquisition. The unique identifiers may subsequently be used to query the available local databases 30A, 30B, 30C for supplementary information SI having the same unique identifier. The supplementary information SI may comprise information pertaining to the disease type and state of the patient, further patient information such as age or sex, the patient's health record, therapy and medication information, information about the practicing physician or the like. The supplementary information SI may be provided in the form of an electronic medical record (EMR), for instance. The local storage devices 30A, 30B, 30C may be part of hospital information systems (HIS), radiology information systems (RIS), clinical information systems (CIS), laboratory information systems (LIS) and/or cardiovascular information systems (CVIS) or the like.

For reviewing genomic data sets by a user, local computing units 40A, 40B, 40C may be configured to execute at least one software component for serving a display unit and a input unit of local computing units 40A, 40B, 40C in order to provide a suited graphical user interface. With the graphical user interface, the user may, for instance, select genomic data sets GDS for review from the acquisition units 50A, 50B, 50C or local storage devices 30A, 30B, 30C. Further, the user may review one or more graphical representations of the genomic data sets GDS as provided by the graphical user interface. Moreover, the graphical user interface may provide the user a selection of analytic tools with which he or she can further analyze the genomic data sets GDS currently under review. Further, the graphical user interface may allow the users to select genomic data sets GDS for sharing with institutions outside of the respective local site A, B, C.

Local computing units 40A, 40B, 40C may be configured to further process the genomic data sets GDS. This may comprise steps such as bringing the genomic data sets GDS into an appropriate format or data compression procedures but may also involve associating and/or appending corresponding supplementary information SI to the genomic data sets GDS as metadata.

Further, in terms of processing the genomic data, local computing units 40A, 40B, 40C may be configured to extract genomic features from the genomic data sets GDS. The genomic features may relate to high-level information derived from the genomic data sets GDS, e.g., by using bio-informatics algorithms. In particular, genomic features may be generated by applying a trained function to the genomic data sets GDS. The trained function may, for instance, be provided by the matching system 1. The trained function may be based on a support vector machine algorithm and/or a random forest algorithm and/or a regularized regression model. The genomic features may be selected or tailored according to the clinical question at hand. For oncology related questions, the genomic features may, for instance, rely on identifying mutations in the genomic data. Accordingly, corresponding genomic features might relate to the genomic regions of mutations, mutation hotspots, the effect of mutations (in terms of gain or loss of function), and/or the clinical actionability of mutations. As an alternative or in addition to that, the preprocessing as described above may also be performed in the acquisition units 50A, 50B, 50C. Moreover, the processing of genomic data sets GDS may comprise a filtering step. For instance, local computing units 40A, 40B, 40C may be configured to filter out information that does not identify a required piece of information such as a chromosomal DNA copy loss or gain. As such, filtered genomic data sets GDS may be created that generally only include those regions that may contain a chromosomal abnormality or alternation.

Thus, summarizing the above, genomic data sets GDS may relate to raw or processed genomic data and may comprise metadata and supplementary information SI. As such, genomic data sets GDS, may, for instance, comprise plain gene sequences, information about gene mutations, gene associations, gain or loss, gene expression levels or gene states, tumor profiles, disease states, sex or age of the patient, and so forth.

The components at the respective sites A, B, C are interfaced with an appropriate local network enabling local communication at the respective sites A, B, C. Data transfer is preferably realized using a network connection. The network may be realized as local area network (LAN), e.g., an intranet, ethernet or a wide area network (WAN). Network connection is preferably wireless, e.g., as wireless LAN (WLAN or Wi-Fi). The network may comprise a combination of the different network types. In particular, the network may comprise a HL7 and/or FHIR compatible network. HL7 (Health Level Seven) specifies a set of flexible standards, guidelines, and methodologies by which various healthcare systems can communicate with each other. It allows information to be shared and processed in a uniform and consistent manner and therefore enables to easily share clinical information. The FHIR (Fast Healthcare Interoperability Resources)-standard builds on previous standards from HL7 and uses a web-based suite of API-technology. It is meant to enhance the interoperability and support a wider variety of devices from workstations to tablets to smart phones.

For patient privacy reasons, there is preferably no direct communication across the different sites A, B, C, however. This restriction is indicated by the dashed lines in Fig. 1. To still enable an exchange across the sites A, B, C, local computing units 40A, 40B, 40C are given the opportunity to upload genomic data sets GDS to the external matching system 1. These uploaded genomic data sets are subsequently assigned the reference numeral GDS1. The computing units 40A, 40B, 40C may comprise an upload module (not shown) interfaced to the matching system 1 via an appropriate network such as an internet connection using, for instance, https-protocols. Upload module and/or matching system 1 may be configured such that only single-directional communication between local computing units 40A, 40B, 40C and the matching system 1 is possible in the sense that local computing units 40A, 40B, 40C may upload information to matching system 1 but cannot directly access and retrieve data from matching system 1. The upload module may function to allow users to upload genomic data sets GDS selected by the user (e.g., via the computing system's user interface) to the matching system 1. Local computing units 40A, 40B, 40C and/or matching system 1 may be configured such that raw genomic data is uploaded to matching system 1. Alternatively, already processed genomic data may be uploaded. For instance, the uploaded genomic data sets GDS1 may comprise (or consist of) the aforementioned genomic features.

Local computing units 40A, 40B, 40C may comprise filtering modules (not shown) configured to filter out personal patient data from the genomic data sets GDS prior to uploading the genomic data sets GDS (i.e., local computing units 40A, 40B, 40C are configured to anonymize the genomic data sets GDS). In addition to that or as an alternative, also matching system 1 may be configured to anonymize the uploaded genomic data sets GDS1, likewise relying on appropriate filtering modules, for instance.

In the example as shown in Figure 1, matching system 1 is part of one of the local sites B (also referred to as "second site" while sites without matching system 1 are also referred to as "first sites") of distributed environment 100. In other words, matching system 1 of Figure 1 is a local system located at one of the sites A, B, C. Matching system 1 comprises a matching engine 10 for comparing genomic data sets GDS and identifying reference genomic data sets and a database 20 storing a plurality of genomic data sets GDS2 for comparison (also denoted as "second genomic data sets"). Further, matching system 1 comprises an interface unit (not shown) configured to communicate with local computing units 40A, 40B, 40C, e.g., for receiving uploaded genomic data sets and for dispatching a notification NOT once reference genomic data sets have been found. Further matching system 1 may comprise a repository 30B, generally configured to store supplementary information SI associated to the genomic data sets GDS2 stored in database 20. Database 20 and/or the repository 30B may be configured as a local or spread storage. Database 20 is configured to store a plurality (i.e., more than 1.000 or more than 10.000 or more than 100.000) genomic data sets GDS2. These genomic data sets GDS2 are either received locally from the site at which matching system 1 is installed (in the example of Figure 1 this is site B) via corresponding local computing 40B and acquisition units 50B or from other local sites A, C. Database 20 may further be configured to store the supplementary information SI related/associated to the genomic data sets GDS2. Database 20 can include any storage medium or organizational unit for storing and accessing genomic data sets GDS2 and supplementary information SI. Further embodiments can include a plurality of databases and can also include distributed data storage architectures as database 20.

Alternatively, supplementary information SI may be stored in repository 30. Like the genomic data sets GDS2, the supplementary information SI may either be recorded locally at the site B where the matching system 1 resides or come from external sites A, C, e.g., in the form of an appendix to the uploaded genomic data sets GDS1.

Matching engine 10 may comprise a plurality of sub-units 11-14 configured to process genomic data sets GDS1, GDS2 for identifying similar genomic data sets and share this information with the external sites A, C. Matching engine 10 may comprise either a computer/processing unit, a microcontroller or an integrated circuit. Alternatively, matching engine 10 may comprise a real or virtual group of computers like a so called 'cluster' or 'cloud'. Further matching engine 10 may be a server system. The server system may be a central server. Further, matching engine 10 may comprise a memory such as a RAM, e.g., for temporally loading genomic data sets GDS2 from the database for further processing.

Sub-unit 11 is a pre-processing module configured to analyze the uploaded genomic data sets GDS1 (also denoted as first genomic data set), to determine if and which pre-processing steps are required for the further analysis. Further, sub-unit 11 is configured to pre-process the uploaded genomic data set GDS1 accordingly. Analyzing may comprise analyzing the format and information content of the uploaded genomic data sets GDS1. Here, it may be determined, for instance, if the uploaded genomic data set GDS1 comprises raw data and/or already processed data. The outcome of this analysis may then be compared to the system requirements of the matching engine 10. If any discrepancy is detected, further pre-processing steps may be scheduled and carried out for bringing the genomic data sets GDS1 in shape for the subsequent similarity search. The pre-processing steps in general may be of the same kind as mentioned in connection with the processing steps performed by the local computing units 40A, 40B, 40C. This may, in particular, involve the extraction of genomic features. As mentioned, these genomic features may address mutations such as the genomic regions of mutations in the genomic data, mutation hotspots in the genomic data, the effect of mutation in the genomic data (gain or loss), and/or the clinical actionability of mutations in the genomic data set. To derive the genomic features, sub-unit 11 may be configured to apply and execute suited bio-informatics-algorithms, and, in particular, one or more trained functions. Whether the pre-processing is done in the matching engine 10 or already locally at the local sites A, C may vary according to the specific requirements. Sourcing out some or all of the pre-processing steps to the local sites A, C has the benefit of reduced data traffic and enhanced data security. By contrast, centralizing the pre-processing at matching engine 10 may improve compatibility and ensures that the full genomic information is still present at matching engine 10. As yet a further option, pre-processing steps may also be split between matching engine 10 and local computing systems 40A, 40B, 40C.

Sub-unit 12 is a module configured to further process the uploaded genomic data sets GDS1 by searching and identifying reference genomic data sets. Reference genomic data sets are those genomic data sets amongst the genomic data sets GDS2 stored in database 20 that are "similar" to the uploaded genomic data sets GDS1. To identify the reference genomic data sets, sub-unit 12 may be configured to calculate a degree of similarity between the uploaded genomic data set GDS1 and the genomic data sets GDS2 from database 20. As will be further detailed below, sub-unit 12 is preferably configured to do so on the basis of a weighted comparison of distinct characteristic values extracted from the genomic data sets GDS1, GDS2 and/or the genomic features. For a more efficient search for reference genomic data sets, sub-unit 12 may also be configured to analyze any metadata adhered to the genomic data sets GDS1, GDS2. As mentioned, the metadata may comprise an indication (or electronic tag) about the kind of disease linked to the genomic data set. By evaluating this information, sub-unit 12 may, for instance, focus on genomic data sets GDS2 in database 20 having the same indication (or electronic tag) and, hence, belong to the same disease group.

Sub-unit 13 is a module for retrieving supplementary information SI associated with the reference genomic data sets. The supplementary information SI may either be adhered to the genomic data sets GDS2 as metadata or be archived separately in designated databases such as repository 30B. If the supplementary information SI is adhered to the genomic data sets GDS2 in the form of metadata, e.g., in a header or the like, sub-unit 13 may be configured to access, read and process the metadata and retrieve the supplementary information SI directly from the genomic data sets GDS2. Alternatively, sub-unit 13 may be configured to query and retrieve the supplementary information SI from the corresponding repository 30A, e.g., by using an appropriate data identifier. Repository 30A may be separate from database 20 or integrated in database 20. As mentioned, the supplementary information SI may be information concerning the attending physician(s) responsible for the case, information concerning the kind of the disease, treatment information, information about the treatment response or the like.

Sub-unit 14 is a module for enabling information exchange across the sites A, C. In this regard, sub-unit 14 may be configured to dispatch a communication (notification NOT) to the site where the uploaded genomic data set GDS1 came from indicating that a reference genomic data set has been found. In this regard, the distributed environment 100 may be configured such that the notification NOT is displayed at the local computing systems 40A, 40B, 40C. Further, sub-unit 14 may be configured to provide a communication channel CH1, CH2 enabling communication between the site of origin of the uploaded genomic data set GDS1 and the site(s) of origin of the reference genomic datasets. The communication channel CH2 may be such that the respective sites of origin may communicate directly, e.g., via the computing systems 40A, 40B, 40C. In addition to that or as an alternative, the communication channel CH1 may be such that the communication between the sites A, B, C takes place via matching engine 10 (sub-unit 14) as communication node. Further, sub-unit 14 may be configured to include part or all of the retrieved supplementary information SI in the notification.

The designation of the distinct sub-units 11-14 is to be construed by ways of example and not as limitation. Accordingly, sub-units 11-14 may be integrated to form one single unit or can be embodied by computer code segments configured to execute the corresponding method steps running on a processor or the like of the matching engine 10. Each sub-unit 11-14 may be individually connected to other sub-units and or other components of the distributed environment 100 where data exchange is needed to perform the method steps. For example, sub-unit 11 may be connected to the interface units of local computing units 40A, 40B, 40C for receiving the uploaded genomic data sets. Likewise, sub-unit 14 may be directly connected to corresponding interface units of local computing units 40A, 40B, 40C to forward the notification NOT that reference genomic data sets have been found. Further, sub-unit 12 may be directly connected to database 20 and sub-unit 30 may be directly connected to repository 30B. In this regard, database 20 and repository 30B may be activated on a request-base, wherein the request is sent by matching engine 10. Interfaces for data exchange with the matching engine 10 may be realized as hardware- or software-interface, e.g., a PCI-bus, USB or fire-wire. Data transfer is preferably realized using a network connection. The network may be realized as local area network (LAN), e.g., an intranet or a wide area network (WAN). Network connection is preferably wireless, e.g., as wireless LAN (WLAN or WiFi). Further, the network may comprise a combination of different networks.

A computing unit according to the invention may comprise part or all of the matching engine 10. Further, it may comprise part or all of the local computing systems 40A, 40B, 40C at the sites A, B, C. Of note, the layout of the computing unit, i.e., the physical distribution of sub-units is, in principle, arbitrary. For instance, filtering modules for anonymizing genomic data sets GDS may be comprised in local computing units 40A, 40B, 40C and/or in matching system 1. The same holds true for pre-processing modules such as sub-unit 11. Specifically, pre-processing modules may also be comprised in local computing units 40A, 40B, 40C or already in the acquisition units 50A, 50B, 50C.

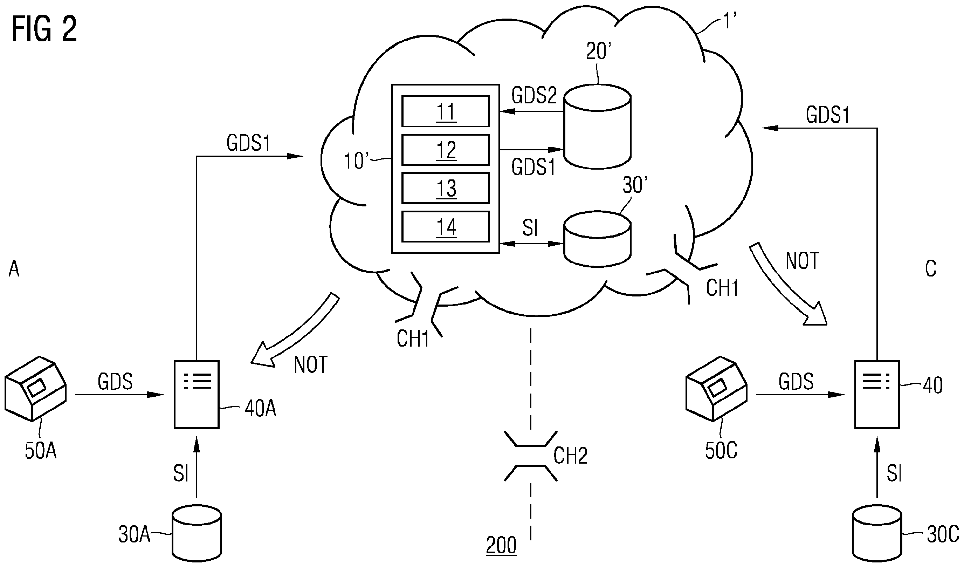

**Figure 2** depicts a distributed environment 200 for sharing medical information according to a second embodiment. With respect to the embodiment described in connection with Figure 1, like reference numerals refer to like parts. In the example shown in Figure 2, two local sites A, B are shown by way of example. This is not to be construed as limiting the disclosure, however. In general, distributed environment 200 may comprise any number of local sites.

One difference between the embodiment shown in Figure 1 and the embodiment shown in Figure 2 is that the matching system 1' is not local in the sense that it is not installed locally at one of the local sites A, B participating in the distributed environment 200. Rather, matching system 1' takes the form of a cloud computing system installed remotely from the local sites A, B. Matching system 1' may comprise a real or virtual group or cluster of computers forming the matching engine 10' and one or more cloud databases forming the database 20' for storing a plurality of genomic data sets GDS2 for comparison and, optionally, a repository 30' for storing supplementary information SI associated with genomic data sets GDS2. Apart from being configured as a cloud computing system, matching engine 10', database 20' and the optional repository 30' are configured identically to the corresponding components of the distributed environment 100 according to the first embodiment. Specifically, matching engine 10' may be configured to comprise like sub-units 11-14 to matching engine 10 and to carry out the same method steps as matching engine 10. In the embodiment according to Figure 2, the local computing systems 40A, 40B could be designated as "frontend" or "client" components facing the user, while matching system 1 might then be conceived as "backend" component. Communication between local computing systems 40A, 40B and matching system 1' may as well be carried out using the https-protocol, for instance. Like in the embodiment shown in Figure 1, the computational power of the system may be distributed between matching system 1' and local computing systems 40A, 40B. In a "thin client" system, the majority of the computational capabilities would exist at the matching system 1'. In a "thick client" system, more of the computational capabilities exist in the local computing systems 40A, 40B. In particular, this applies for the step of pre-processing genomic data sets GDS (e.g., by extracting genomic features) which may take place locally at sites A, B or at the matching system 1'.

As in the case of the embodiment shown in Figure 1, the communication between the matching system 1' and the sites A, B, is configured such that the local computing systems 40A, 40B, albeit capable of uploading genomic data sets to the matching system 1', are not allowed to directly query and retrieve information from matching system 1'. In addition, the system resources of one site are generally not accessible by other sites in the distributed environment 200. This restriction is indicated by the dashed line in Fig. 2.

**Figure 3** depicts a method for identifying a reference genomic data set according to an embodiment of the present invention.

The method comprises several steps. The order of the steps does not necessarily correspond to the numbering of the steps but may also vary between different embodiments of the present invention. The steps subsequently described may be executed by the distributed environment 100 as depicted in Figure 1 as well as by the distributed environment 200 as depicted in Figure 2. If not indicated otherwise steps S10 to S60 are performed by the matching engine 10, 10'. Steps or sub-steps that are optional are shown with a dashed frame.

A first step S10 is directed to receiving an uploaded genomic data set GDS1 at the matching system 1, 1' from one of the sites A, B, C. The site from which the uploaded genomic data set has been uploaded may also be denoted as "first site". As will be further detailed in connection with Figure 4 below, the uploaded genomic data set GDS1 (also denoted as "first genomic data set") has been acquired at one of the local sites A, B, C and is uploaded therefrom. Optionally, step S10 may comprise assigning a suitable unique identifier to the uploaded genomic data set (if not already provided for by the local sites). The unique identifier is configured such that the uploaded genomic data sets GDS1 are traceable in matching system 1, 1'. It may comprise an accession number and/or encode from which site the uploaded genomic data set has been uploaded. Step S10 may comprise the optional sub-step S11 of extracting metadata or supplementary information SI from the uploaded genomic data set GDS1. For instance, this supplementary information SI may then be used as context information for comparing the uploaded genomic data set GDS1 to the genomic data sets GDS2 stored in database 20, 20'. Further, step S10 may comprise the optional sub-step S12 of analyzing the uploaded genomic data sets GDS1. In this step, it may generally be determined which format the uploaded genomic data sets GDS1 have (i.e., whether or not filtering and/or feature extraction has been applied to the uploaded genomic data set) and, accordingly, which (pre-)processing steps are required for the ensuing similarity search. It may further be determined in sub-step S12, whether or not the uploaded genomic data set GDS1 has already been anonymized. If not, this is preferably done before further processing the uploaded genomic data set GDS1 to ensure that patient privacy is not violated. To anonymize the uploaded genomic data set GDS1, step S10 may comprise an optional sub-step S13 for filtering out any personal information from the uploaded genomic data sets GDS1.

Subsequently, in step S20, the uploaded genomic data set GDS1 is compared to a plurality of genomic data sets GDS2 stored in database 20, 20' (also denoted as "second genomic datasets"). The step of comparing may comprise accessing database 20, 20' and retrieving one or more genomic data sets GDS2 from database 20, 20' for comparison. The comparison may be carried out with respect to all of the genomic data sets GDS2 stored in database 20, 20' or just with respect to a subset of the genomic data sets GDS2. Specifically, matching engine 10, 10' may be configured to preselect one or more genomic data sets GDS2 from database 20, 20' so that the uploaded genomic data set GDS1 is only compared to a fraction of the genomic data sets GDS2 comprised in database 20, 20'. In Figure 3, this preselection is shown as an optional sub-step S21. The preselection may be based on matching supplementary information SI of the uploaded genomic data set GDS1 with corresponding supplementary information SI associated to the genomic data sets GDS2. For instance, genomic data sets GDS2 may be preselected for comparison if they fall in the same disease group as the uploaded genomic data set GDS1. A disease group may relate to cases having a clinical and/or functional similarity of the underlying diseases. Self-speaking also further factors may be considered in this regard. According to an example, the genomic data sets GDS2 may also be preselected according to tumor types or gene alternations, for instance.

In subsequent step S30, one or more reference genomic data sets are identified based on the genomic data sets GDS2 selected for comparison. As mentioned, a reference genomic data set is a genomic data set which has a certain degree of similarity to the uploaded genomic data set GDS1. The identification of similar genomic data sets may be based on the genomic sequence as such or, in other words, on raw data. In this regard, there are several known ways. One involves evaluating a spatial overlap of the gene sequences. However, according to several embodiments, the comparison is based on one or more higher-level genomic features or characteristic values $CV1...CVn$ encoded in the gene sequence that - dependent on the state of the genomic data sets - might require further processing of the genomic data sets. These genomic features or characteristic values $CV1...CVn$ correspond to so called "similarity criteria". The similarity criteria may be chosen according to the case and/or the genomic data set at hand. In cancer therapy, the analysis of mutations in the gene sequence plays an important role and, accordingly, similarity criteria may likewise be based on evaluating mutations in the gene sequence. The corresponding genomic and/or characteristic values $CV1...CVn$ may relate to very specific characteristics, such as the exact location of a given mutation in the gene sequence, but may as well concern more generic characteristics, such as the effect of mutations in the signaling pathway.

Exemplary similarity criteria include

- the genomic region of a mutation,
- the presence of a mutation hotspot (are mutations occurring within a window of a predefined sequence length of amino acids?),
- the clinical actionability of mutations,
- the mutation consequence (e.g., gain vs. loss of function), or
- the effect of mutations on signaling pathways.

As regards the clinical actionability, the ESMO Scale for Clinical Actionability of molecular Targets (ESCAT) may be used, for instance. Alternatively, the clinical actionability may be determined according to the guidelines of the Association for Molecular Pathology (AMP).

Each genomic feature may correspond to one or more characteristic values CV1...CVn. In this regard, the genomic features may be considered as a more abstract form of features extracted from a gene sequence as compared to the characteristic values CV1...CVn. Genomic features may relate to data objects which can be translated into one or more characteristic values CV1...CVn.

For identifying similarities among two genomic data sets, a degree of similarity may be determined by comparing the individual genomic features and/or characteristic values CV1...CVn. Taking the genomic region of a mutation as an example, such an assessment may involve extracting the genomic region of a given mutation from the gene sequence of the uploaded genomic data set GDS1, extracting the corresponding genomic region from the gene sequence of a stored genomic data set GDS2, and comparing the ensuing characteristic values CV1...CVn, e.g., in the form of calculating the difference in characteristic values CV1...CVn. The result provides an indication of whether or not a mutation is at the same position in two genomic data sets GDS1, GDS2. Evidently, the result may be improved by sampling not only one similarity criterion but a plurality of different criteria. The ensemble of genomic features and/or characteristic values CV1...CVn characterizes a genomic data set GDS1, GDS2 and, hence, may be used to efficiently identify similar genomic data sets. Such an ensemble may also be denoted as a genomic feature vector or feature set.

The genomic features and/or characteristic values CV1...CVn may be extracted from the respective genomic data sets GDS1, GDS2 upon the actual identification of one or more reference genomic data sets, i.e., in the framework of step S30. In this case, step S30 may comprise an optional sub-step S31 in the form of a pre-processing step of extracting on or more genomic features and/or characteristic values CV1...CVn from the genomic data sets GDS1, GDS2 according to one or more similarity criteria. According to an embodiment, step S31 involves applying the aforementioned trained function to the uploaded genomic data set GDS1 and/or the genomic data sets GDS2 from database 20, 20'. This pre-processing step S31 is optional, however, and may depend on the state of the uploaded genomic data set GDS1 (as, for instance determined in optional step S12), the state of the genomic data sets GDS2 as stored in database 20, 20' and the actual method relied upon for identifying similar genomic data sets. As an alternative and as already explained previously, the extraction of one or more genomic features and/or characteristic values CV1...CVn may also be carried out in the framework of previous steps S10 or S20. What is more, at least the genomic data sets GDS2 comprised in database 20, 20' may be held available in an already pre-processed format with the genomic features and/or characteristic values CV1...CVn already extracted and disposable. A corresponding pre-processing is preferably performed upstream of the actual steps for identifying one or more reference genomic data sets GDS2 as this reduces the computation time for each uploaded genomic data set GDS1. For instance, the feature extraction may be carried out when integrating new genomic data sets GDS2 into database 20, 20'. As mentioned, the extraction of the genomic features according to a set of similarity criteria may furthermore already be carried out at the local sites A, B, C (e.g., in the local computing units 40A, 40B, 40C). Other procedures that may form part of a pre-processing step (either within or outside of step S31) may include filtering out irrelevant information from the genomic data sets. For instance, portions of the sequence may be filtered out that do not identify a chromosomal DNA copy loss or gain. As such, filtered genomic data sets may be generated that generally only include those regions that may contain a chromosomal abnormality. Like in the case of the genomic feature extraction, this pre-processing may be performed already at the local sites A, B, C or by the matching system 1, 1' once a genomic data set GDS1 has been uploaded. For the actual identification of one or more reference genomic data sets, a similarity between the genomic data sets GDS1, GDS2 needs to be quantified. This may, for instance, be done by combining the genomic features of the involved genomic data sets GDS1, GDS2 to form feature vectors. A degree of similarity may then be derived by calculating the dot product between the feature vector of the uploaded genomic data set GDS1 and the corresponding feature vector of genomic data set GDS2 from database 20, 20' (also referred to as "cosine similariy"). Alternatively, a sum of squared differences between genomic features and/or characteristic values of two genomic data sets GDS1, GDS2 may be calculated as measure for the similarity. Further alternatively, the genomic features and/or characteristic values CV1...CVn may be aggregated to a score S for each genomic data set GDS1, GDS2. Specifically, the score S may be defined as the weighted sum of a plurality of genomic features and/or characteristic values CV1...CVn as follows:

$$S = W1*CV1 + W2*CV2 + \ldots + Wn*CVn.$$

In the above formula, WI...Wn denote weights, which may be positive or negative. Generally speaking, the weights W1...Wn may be seen as indicating the importance of the corresponding genomic feature and/or characteristic value CV1...CVn for finding similar genomic data sets GDS2. The degree of similarity between two genomic data sets GDS1, GDS2 may then be expressed as the difference or distance in the corresponding scores S. Of note, also the summands in the abovementioned dot product or the sum of the squared differences may be correspondingly weighted.

According to an embodiment, all or part of the procedures taking place in step S30 might be performed by one or more trained functions (which are applied on the uploaded genomic data set GDS1 and or the genomic data sets GDS2 in database 20, 20'). According to the above, the trained functions may thus be configured (trained) so as to extract genomic features from the genomic data sets GDS1, GDS2 and output them either as intermediate values or their final output, to score the genomic data sets GDS1, GDS2 on the basis of the genomic features and/or to deliver one or more reference genomic data sets on that basis. However, the trained functions may also follow a completely different procedure and may just indicated one or more reference genomic data sets as the final result. The trained function may be based on regularized regression models (e.g. lasso, elastic net etc.), random forest algorithms, and/or support vector machines.

Once the similarity between the uploaded genomic data set and the genomic data sets GDS2 stored in database 20, 20' has been quantified in terms of the degree of similarity, one or more reference genomic data sets may be identified on that basis. This may involve ranking the genomic data sets GDS2 according to their degree of similarity to the uploaded genomic data set GDS1. The genomic data sets GDS2 ranked highest may then be identified as reference genomic data set(s). As an alternative or in addition to that, the degrees of similarity may be compared to a predefined threshold. Genomic data sets GDS2 with degrees of similarity above the predefined threshold may then be selected as reference genomic data set. The predefined threshold may be set automatically and/or (semi-)automatically and/or by a user. If none of the genomic data sets GDS2 has a degree of similarity greater than the predefined threshold, either no reference genomic data set is identified at all or the genomic data set(s) GDS2 with the highest degree of similarity is identified as reference genomic data set(s). Further, the identification of reference genomic data sets amongst the second genomic data sets may involve selecting those second genomic data sets as reference genomic data sets, the score of which lies within a predetermined margin around the score of the first genomic data set. The predetermined margin may be set automatically and/or (semi-)automatically and/or by a user.

A further step S40 is directed to dispatching a notification NOT to the site from which the uploaded genomic data set GDS1 has been uploaded. Notification NOT may be indicative, in general, of the result of the genomic similarity search performed by matching system 10, 10'. According to an embodiment, notification NOT may be indicative of the one or more reference genomic data sets identified. If no reference genomic data set could be identified, this may be included in notification NOT as well. Optionally, step S40 may include sub-step S41 of retrieving, for each reference genomic data set, supplementary information SI and adhering it to the notification NOT. The supplementary information SI may include contact information of the attending physician, information about the therapy and the therapy response, or the like. As mentioned, the supplementary information SI may either be already comprised in the reference genomic data sets or be archived separately in designated databases such as in EMR- repositories 30B, 30'. Accordingly, the supplementary information SI may directly be retrieved from the reference genomic data sets or by querying corresponding repositories 30B, 30' (e.g., based on the aforementioned unique identifiers).

Optional step S50 is directed to importing the uploaded genomic data set GDS1 into the matching system 1, 1'. This may comprise storing the uploaded genomic data set GDS1 in database 20, 20' and archiving any supplementary information SI associated to the uploaded genomic data set GDS1 (e.g., either in database 20 itself or in repository 30B, 30'). Upon importing, the uploaded genomic data set GDS1 may be formatted such as to correspond to the genomic data sets GDS2 already stored in database 20, 20'. This may comprise extracting genomic features and/or characteristic values CV1...CVn according to the one or more similarity criteria from the uploaded genomic data set GDS1. Further, data import may also include automated operations of tagging data as well as mapping the imported data to data already archived in the system. The actions of tagging and mapping may be based on any metadata adhered to the uploaded genomic data set and/or any piece of supplementary information SI uploaded together with the uploaded genomic data set. For instance, the disease type may be extracted from either the metadata or the supplementary information SI and used to map the uploaded genomic data set to a disease group within database 20, 20'. Prior to archiving, the uploaded genomic data set and any supplementary information SI may be subjected to an appropriate filtering procedure in order to ensure that the archived data is anonymized.

A further optional step S60 is directed to create a communication channel CH1, CH2 between the sites associated with the matched genomic data sets. The communication channel CH2 may be configured such that it facilitates direct communication between the treating physicians associated with the matched genomic data sets GDS1, GDS2. In one embodiment, the communication channel CH1, CH2 is configured such that the communication is anonymous without the need to identify a specific patient and/or physician. The communication may, for instance, be effected via the local computing systems 40A, 40B, 40C. In this regard, the communication channel CH2 may connect local computing units 40A, 40B, 40C directly, e.g., by ways of a secure internet connection. Alternatively, the communication channel CH1 may be such that the communication is routed via the matching system 1, 1'. In other words, the matching system 1, 1' takes the role of a connectivity node between the local sites A, B, C associated with the matched genomic data sets. In addition to that or as an alternative, the communication channel CH1, CH2 may enable a selective access to database 20, 20' and/or the repository 30A, 30' of the matching system 1, 1'. Further, the communication channel CH1, CH2 may be configured such that it enables local sites A the selective (one-time) access to a corresponding database 30C of another site. Further, the communication channel CH1, CH2 may be such that it provides the local site which uploaded a genomic data set GDS1 supplementary information SI for download. To this end, a URL may be provided to the respective local sites, via which the data can be accessed and downloaded. The URL may, for instance, be included in the notification NOT. Further, the communication channel CH1, CH2 may be configured such that it induces local sites A to forward supplementary information SI associated to the one or more reference genomic data sets to the site of origin of the uploaded genomic data set GDS1.

**Figure 4** depicts a method for identifying a reference genomic data set according to an embodiment of the present invention. The method comprises several steps. The order of the steps does not necessarily correspond to the numbering of the steps but may also vary between different embodiments of the present invention. The steps subsequently described may be executed by the distributed environment 100 as depicted in Figure 1 as well as by the distributed environment 200 as depicted in Figure 2. If not indicated otherwise, steps S1 to S8 are performed at the local sites A, B, C, e.g., by local computing units 40A, 40B, 40C and/or the acquiring systems 50A, 50B, 50C. Steps or sub-steps that are optional are shown with a dashed frame in Figure 4.

A first step S1 is directed to acquire genomic data sets GDS by acquisition units 50A, 50B, 50C. This may involve collecting a patient sample and inferring the genetic sequence from it (sequencing). Of note, the sequencing may be obtained from different cells of the body, for example, cells from a tumor. At this stage, the genomic data set GDS may mainly comprise genomic raw data such as a complete genetic sequence of the human genome, or one or more partial genetic sequences, for example, of a chromosome or part of a chromosome. Genetic information included within the genomic data set GDS may include nucleic acids, such as DNA or RNA, coding and/or noncoding RNA expression, and any other genetic or epigenetic modifications such as acetylations, methylations, or others. Further, acquisition units 50A, 50B, 50C may be configured to include metadata in the genomic data set GDS such as a patient ID, patient sex and/or age, the attending physician, a case number or the like. Moreover, the genomic data sets GDS may be provided with an unique identifier in the form of a data tag making the genomic data set GDS unambiguously identifiable at least within the local sites A, B, C. The unique identifier may be a local accession number, for instance. Preferably, the unique identifier is furthermore indicative of the local site A, B, C at which the genomic data set GDS has been generated making the genomic data set GDS traceable to the respective site A, B, C.

A second optional step S2 is directed to pre-process the genomic data set GDS. This may comprise filtering the genomic data set GDS for relevant information. For instance, the raw data may be filtered for gene sequences containing abnormalities and/or mutations which may be meaningful for the later comparison to other genomic data sets GDS2. Moreover, the pre-processing step may comprise evaluating the raw genomic data set GDS according to one or more similarity criteria for the later similarity search in the matching system 1, 1'. The evaluation of the similarity criteria may yield an associated genomic feature or feature set and/or corresponding characteristic values CV1...CVn. The ensemble of genomic features and/or characteristic values CV1...CVn characterizes the genomic data set GDS for a given clinical question. The ensemble of genomic features may be appended to the raw data contained in the genomic data set GDS. According to an embodiment, the ensemble of genomic features and/or characteristic values CV1...CVn may take the place of the raw data in the genomic data set GDS so that the genomic data set GDS only contains processed data in the form of the genomic features. According to an embodiment, step S2 is performed at local computing units 40A, 40B, 40C. As an alternative, at least parts of step S2 may also be performed already upon acquiring the genomic data set at the acquiring units 50A, 50B, 50C.

A further optional step S3 is directed to retrieve supplementary information SI corresponding to the genomic data set GDS and adhere it the genomic data set GDS. This may involve querying local databases 30A, 30B, 30C at the sites A, B, C for supplementary information SI. This may be done using the aforementioned unique identifiers unambiguously linking the respective genomic data set GDS to the supplementary information SI. As mentioned, the supplementary information SI may include context information for the genomic data set GDS which may prove helpful for the later comparison to other genomic data sets GDS2 in the matching system 1, 1'. This may include annotated genes, features, physiological measurements, patient medical history, and/or phenotypic disease descriptions. According to an embodiment, step S3 is performed at local computing units 40A, 40B, 40C.

A further optional step S4 is directed to selecting a genomic data set GDS1 for uploading it to the matching system 1, 1'. To this end, the respective genomic data set GDS may be presented to a user via a graphical user interface at the local computing systems 40A, 40B, 40C. The user may then manually select whether or not the genomic data set GDS shall be uploaded to the matching system 1, 1' for retrieving similar cases. To assist the user in this decision, local computing units 40A, 40B, 40C may be configured to display supplementary information SI corresponding to the genomic data set GDS under consideration. As an alternative, step S4 may also comprise a semi-automatic selection or automated pre-selection of uploading candidates which may, for instance, be based on prior actions of the user and may be presented to the user for review. Moreover, step S4 may comprise a fully automatic selection of genomic data set GDS for uploading. As mentioned, step S4 is optional. This may mean that the distributed environment 100, 200 may also be configured such that all genomic data sets GDS generated are (automatically) uploaded to the matching system 1, 1'. According to an embodiment, step S4 is performed at local computing units 40A, 40B, 40C.

Another optional step S5 is directed to anonymize the genomic data set GDS1 selected for upload. This may comprise filtering out any personal information from genomic data set GDS1 that would enable identifying the patient belonging to genomic data set GDS1. According to an embodiment, step S5 is performed at local computing units 40A, 40B, 40C.

A further step S6 is directed to uploading the genomic data set GDS1 to the matching system 1, 1'. This may be performed using mutual interfaces (in the form of one or more interface units) at the local sites A, B, C and the matching system 1, 1'. The upload may be effected via internet connection using an appropriate protocol such as https. According to an embodiment, step S6 is performed/initiated at local computing units 40A, 40B, 40C.

A further step S7 is directed to receiving notification NOT, e.g., via the mutual interfaces at the local sites A, B, C and the matching system 1, 1'. Notification NOT may indicate to a user that a reference genomic data set and therewith a similar case has been found by the matching system 1, 1'. Upon receipt, notification NOT may be displayed to the user via an appropriate graphical user interface at local computing units 40A, 40B, 40C. Notification NOT may contain supplementary information SI associated to the reference genomic data set such as phenotypic disease information, information about treatment and treatment response, disease progression, contact information about the attending physician. If no reference genomic data set has been found, this may likewise be indicated in notification NOT.

Another optional step S8 is directed to permitting communication via a communication channel CH1, CH2 between the local sites A, C, B associated to the matched genomic data sets. For instance, a communication session may be conducted between physicians associated to the matched genomic data sets via an appropriate communication channel CH1, CH2 as provided for by matching system 10, 10'. As mentioned, the communication channel CH2 may be either be established as a direct link between the involved sites A, B, C or routed through the matching system 1, 1' (communication channel CH1). The communication channel CH1, CH2 may be a communication platform, e.g., chat room for exchanging text messages or a video conference platform. If the communication is routed through the matching system 1, 1', the matching system 1, 1' may be configured to host such a communication platform. What is more, communication may also include that the matched sites are granted mutual access to their databases for retrieving supplementary information SI associated with the matched genomic data sets. For data privacy reasons, this access is preferably selective in the sense that only information relevant to the case at hand may be accessed and that the accessible information is anonymized. In addition to that or as an alternative, the supplementary information SI associated to the matched genomic data sets may be provided by the matching system 1, 1' at a designated repository which may be accessed by the local sites A, B, C for download.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments.

The following points are also part of the disclosure:

1. Computer-implemented method for sharing medical information in a distributed environment comprising a plurality of local sites, the method comprising the steps of:

- receiving a first genomic data set, the first genomic data set being generated at a first one of the local sites, wherein the first genomic data set comprises genomic data of a first patient;
- comparing the first genomic data set with a plurality of second genomic data sets stored in a database external to the first site, wherein the second genomic data sets respectively comprise genomic data of patients different than the first patient;
- identifying, amongst the second genomic data sets, one or more reference genomic data sets, on the basis of determining a similarity between the first genomic data set and the second genomic data sets, the reference genomic data sets having a predetermined degree of similarity to the first genomic data sets;
- dispatching a notification to the first site indicative of the one or more reference genomic data sets.

2. Method according to 1, wherein the first and second genomic data sets do not comprise any personal information of the corresponding patient.

3. Method according to any of the preceding points, wherein at least a portion of the second genomic data sets has been generated at local sites different than the first site.

4. Method according to any of the preceding points, wherein the database is configured such that it cannot be accessed by the first site.

5. Method according to any of the preceding points, wherein the steps of receiving, comparing, identifying, and dispatching are carried out externally to the first site.

6. Method according to any of the preceding points, further with the step of including (or incorporating) the first genomic data set in the database.

7. Method according to any of the preceding points, wherein
the first genomic data set comprises one or more genomic features respectively derived from an underlying gene sequence of a patient at the first site; and
the step of identifying is based on the one or more genomic features.

8. Method according to any of the preceding points, wherein
the first genomic data set consists of one or more genomic features respectively derived from an underlying genetic sequence of a patient at the first site; and
the step of identifying is based on the one or more genomic features.

9. Method according to 7 or 8, wherein the genomic features are based on evaluating mutations in the underlying genetic sequence, wherein the genomic features preferably comprise one or more genomic regions of mutations in the underlying genetic sequence; one or more mutation hotspots in the underlying genetic sequence; one or more effects of mutation in the underlying genetic sequence; and/or one or more clinical actionabilities of mutations in the underlying genetic sequence.

10. Method according to 7, 8 or 9, wherein the step of identifying comprises comparing the genomic features of the first genomic data set with corresponding genomic features of the second genomic datasets.

11. Method according to 8 to 10, further with the step of extracting one or more genomic features from first and/or second genomic datasets.

12. Method according to 11, wherein the step of extracting is based on applying a trained function to the first and/or second genomic data set, wherein the trained function is preferably based on a support vector machine algorithm and/or a random forest algorithm and/or a regularized regression model.

13. System for sharing medical information in a distributed environment comprising a plurality of local sites, the system comprising:

◦ an interface unit configured to communicate with at least one first site of the local sites for receiving a first genomic data set comprising genomic data of a first patient;
◦ a database external to the first site, the database being configured to store second genomic data sets, the second genomic data sets respectively comprising genomic data of second patients different than the first patient;
◦ a computing unit external to the first site and configured to

- receive the first genomic data set;
- retrieve a plurality of second genomic data sets from the database;
- compare the first genomic data sets with the plurality of second genomic data sets;
- identify, amongst the plurality of second genomic data sets, one or more reference genomic data sets, on the basis of determining a similarity between first genomic data set and the second genomic data sets, the reference genomic data sets having a predetermined degree of similarity to the first genomic data set;
- dispatching a notification to the first site indicative of the reference genomic data sets via the interface unit.

14. Usage of the method according to any one of points 1 to 12 for identifying one or more patients having a similar genomic data set as compared to the first patient.

15. Method for sharing medical information comprising the steps of:

- receiving a first genomic data set, the first genomic data set being generated at a first site;
- comparing the first genomic data sets with a plurality of second genomic data sets stored in a database external to the first site;
- calculating, for each of the second genomic data sets, a degree of similarity to the first genomic data set;
- identifying, amongst the second genomic data sets, reference genomic data sets on the basis of the calculated degrees of similarity;

dispatching a notification to the first site indicative of the reference genomic data sets.

**Claims**

1.  Computer-implemented method for sharing medical information comprising the steps of:

    • receiving (S10) a first genomic data set (GDS1), the first genomic data set (GDS1) being generated at a first site (A, C);
    • comparing (S20) the first genomic data sets (GDS1) with a plurality of second genomic data sets (GDS2) stored in a database (20, 20') external to the first site (A, C);
    • identifying (S30), amongst the second genomic data sets (GDS2), one or more reference genomic data sets, on the basis of determining a similarity between the first genomic data set (GDS1) and the second genomic data sets (GDS2) ;
    • dispatching (S40) a notification (NOT) to the first site (A, C) indicative of the one or more reference genomic data sets.

2.  Method according to claim 1, further with the steps of:

    determining one or more similarity criteria associated with the first and second genomic data sets (GDS1, GDS2), preferably based on the first genomic data set (GDS1), preferably based on a gene mutation of the first genomic data set (GDS1);
    processing (S31) the first and second genomic data sets (GDS1, GDS2) so as to respectively extract, from the first and second genomic data sets (GDS1, GDS2), one or more characteristic values (CV1...CVN) respectively corresponding to the one or more similarity criteria; and
    identifying the one or more reference genomic data sets on the basis of the characteristic values (CV1...CVN).

3.  Method according to claim 2, wherein processing of the first genomic data set (GDS1) so as to extract, from the first data set (GDS1), the one or more characteristic values (CV1...CVN) is performed at the first site (A, C).

4.  Method according to claims 2 or 3, wherein the one or more similarity criteria comprise at least one of:

    genomic regions of mutations in the genomic data sets;
    mutation hotspots in the genomic data sets;
    effect of mutation in the genomic data sets;
    clinical actionability of mutations in the genomic data sets;
    tumor profiles;
    disease types;
    patient's age and/or sex;
    treatment plan and/or treatment response; and/or
    any combination thereof.

5.  Method according to any of claims 2 to 4, wherein the step of identifying (S30) comprises:

    calculating, for the first and second genomic data sets (GDS1, GDS2), a score (S) as the weighted sum of the respective characteristic values (CV1...CVN); and

comparing the scores (S) of first and second genomic data sets (GDS1, GDS2); and

preferably, selecting, amongst the second genomic data sets (GDS2) those second genomic data sets as reference genomic data sets the score (S) of which is similar to the score (S) of the first genomic data set (GDS1), preferably the score (S) of which corresponds to the score (S) of the first genomic data set (GDS1) within a predetermined margin.

6. Method according to any of the preceding claims, wherein

the first genomic data set (GDS1) comprises one or more genomic features respective derived from an underlying genetic sequence of a patient; and

the step of identifying (S30) is based on the one or more genomic features.

7. Method according to any of the preceding claims, wherein the step of identifying (S30) comprises generating a ranking of the reference genomic data sets on the basis of their similarity to the first genomic data set (GDS1).

8. Method according to any of the preceding claims, wherein the step of dispatching (S40) further comprises the step of retrieving (S41), for each reference genomic data set, supplementary information (SI); and

including the supplementary information (SI) in the notification (NOT);

the supplementary information preferably comprising at least one of:

- a contact information associated with the respective reference genomic data set;
- an information at which sites the with the respective reference genomic data set has been generated;
- a therapy history associated e with the respective reference genomic data set;
- a treatment response profile associated with the respective reference genomic data set;
- a genetic tumor profile associated with the respective reference genomic data set; and/or
- any combination thereof.

9. Method according to any of the preceding claims, further comprising a step of establishing (S60) a communication channel (CH1, CH2) for communication between the first site (A, C) and the respective sites of origin of the one or more reference genomic data sets.

10. Method according to any of the preceding claims, wherein

the database (20) is a local database located at a second site (B) different than the first site (A, C);

in the step of receiving (S10), the first genomic data set (GDS1) is received at the second site (B); and

the steps of comparing (S20), identifying (S30) and dispatching (S40) are carried out at the second site (B).

11. Method according to any of claims 1 to 9, wherein

the database (20') is configured as a cloud platform;

in the step of receiving (S10), the first genomic data set (GDS1) is received at the cloud platform; and

the steps of comparing (S20), identifying (S30) and dispatching (S40) are carried out at the cloud platform.

12. Method according to any of the preceding claims, wherein the step of identifying (S30) is based on applying a trained function to the first genomic data set (GDS1), preferably to the first and the second genomic data sets (GDS1, GDS2), wherein the trained function is preferably based on a support vector machine algorithm and/or a random forest algorithm and/or a regularized regression model.

13. System (1, 1') for sharing medical information comprising:

◦ an interface unit configured to communicate with a first site (A, C) for receiving a first genomic data set (GDS1) from the first site;

◦ a database (20, 20') storing second genomic data sets (GDS2), the database (20, 20') being external to the first site;

◦ a computing unit (10, 10') external to the first site configured to:

- receive the first genomic data set (GDS1) via the interface unit;
- retrieve a plurality of second genomic data sets (GDS2) from the database for comparison with the first genomic data set (GDS1)
- compare the first genomic data set (GDS1) with the plurality of second genomic data sets (GDS2);
- identify, amongst the plurality of second genomic data sets (GDS2), one or more reference genomic data sets, on the basis of determining a similarity between the first genomic data set (GDS1) and the second genomic data sets (GDS2);
• dispatching a notification (NOT) to the first site (A, C) indicative of the reference genomic data sets via the interface unit.

14. Computer program product comprising program elements which induce a computing unit of a system for sharing medical information to perform the steps according to the method of any of claims 1 to 12, when the program elements are loaded into a memory of the computing unit.

15. Computer-readable medium on which program elements are stored that are readable and executable by a computing unit of a system for sharing medical information, in order to perform steps of the method according to any of claims 1 to 12, when the program elements are executed by the computing unit.

FIG 1

EP 3 799 051 A1

FIG 2

FIG 3

```
┌──────────────────────┐
│                      │──S10
└──────────┬───────────┘
           │        ┌ ─ ─ ─ ─ ─ ┐
           │          ─S11
           │        └ ─ ─ ─ ─ ─ ┘
           │        ┌ ─ ─ ─ ─ ─ ┐
           │          ─S12
           │        └ ─ ─ ─ ─ ─ ┘
           │        ┌ ─ ─ ─ ─ ─ ┐
           │          ─S13
           ▼        └ ─ ─ ─ ─ ─ ┘
┌──────────────────────┐
│                      │──S20
└──────────┬───────────┘
           │        ┌ ─ ─ ─ ─ ─ ┐
           │          ─S21
           ▼        └ ─ ─ ─ ─ ─ ┘
┌──────────────────────┐
│                      │──S30
└──────────┬───────────┘
           │        ┌ ─ ─ ─ ─ ─ ┐
           │          ─S31
           ▼        └ ─ ─ ─ ─ ─ ┘
┌──────────────────────┐
│                      │──S40
└──────────┬───────────┘
           │        ┌ ─ ─ ─ ─ ─ ┐
           │          ─S41
           ▼        └ ─ ─ ─ ─ ─ ┘
┌ ─ ─ ─ ─ ─ ─ ─ ─ ─ ─ ┐
                      ──S50
└ ─ ─ ─ ─ ─┬─ ─ ─ ─ ─ ┘
           │
           ▼
┌ ─ ─ ─ ─ ─ ─ ─ ─ ─ ─ ┐
                      ──S60
└ ─ ─ ─ ─ ─ ─ ─ ─ ─ ─ ┘
```

FIG 4

```
┌──────────────────────┐
│                      │──S1
└──────────┬───────────┘
           ▼
┌ ─ ─ ─ ─ ─ ─ ─ ─ ─ ─ ┐
                      ──S2
└ ─ ─ ─ ─ ─┬─ ─ ─ ─ ─ ┘
           ▼
┌ ─ ─ ─ ─ ─ ─ ─ ─ ─ ─ ┐
                      ──S3
└ ─ ─ ─ ─ ─┬─ ─ ─ ─ ─ ┘
           ▼
┌ ─ ─ ─ ─ ─ ─ ─ ─ ─ ─ ┐
                      ──S4
└ ─ ─ ─ ─ ─┬─ ─ ─ ─ ─ ┘
           ▼
┌ ─ ─ ─ ─ ─ ─ ─ ─ ─ ─ ┐
                      ──S5
└ ─ ─ ─ ─ ─┬─ ─ ─ ─ ─ ┘
           ▼
┌──────────────────────┐
│                      │──S6
└──────────┬───────────┘
           ▼
┌──────────────────────┐
│                      │──S7
└──────────┬───────────┘
           ▼
┌ ─ ─ ─ ─ ─ ─ ─ ─ ─ ─ ┐
                      ──S8
└ ─ ─ ─ ─ ─ ─ ─ ─ ─ ─ ┘
```

Europäisches
Patentamt

European
Patent Office

Office européen
des brevets

# EUROPEAN SEARCH REPORT

**Application Number**

EP 19 20 0381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KE CHENG ET AL: "Secure Similar Sequence Query on Outsourced Genomic Data", PROCEEDINGS OF THE 2018 ON ASIA CONFERENCE ON COMPUTER AND COMMUNICATIONS SECURITY , ASIACCS '18, 1 January 2018 (2018-01-01), pages 237-251, XP055641755, New York, New York, USA DOI: 10.1145/3196494.3196535 ISBN: 978-1-4503-5576-6 * page 239 left col last par - page 239 right col par 3, page 240 left col par 1, fig 1 * | 1-15 | INV. G16B40/10 G16B50/20 |
| A | US 2016/070859 A1 (IGNATENKO TANYA [NL]) 10 March 2016 (2016-03-10) * par 34, 37-38, 41 * | 1-15 | |

TECHNICAL FIELDS
SEARCHED      (IPC)

G16B

The present search report has been drawn up for all claims

| Place of search | Date of completion of the search | Examiner |
|---|---|---|
| Munich | 31 March 2020 | Bankwitz, Robert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
....................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 20 0381

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2020

| Patent document cited in search report | Publication date | Patent family member(s) | | Publication date |
|---|---|---|---|---|
| US 2016070859 A1 | 10-03-2016 | CN | 105229651 A | 06-01-2016 |
| | | EP | 3000067 A2 | 30-03-2016 |
| | | JP | 6373977 B2 | 15-08-2018 |
| | | JP | 2016524749 A | 18-08-2016 |
| | | US | 2016070859 A1 | 10-03-2016 |
| | | WO | 2014188290 A2 | 27-11-2014 |

EPO FORM P0459

For more details about this annex : see Official Journal of the European Patent Office, No. 12/82